# EUROPEAN PATENT SPECIFICATION

(11) **EP 0 455 607 B1**
(45) Date of publication and mention of the grant of the patent: **12.07.1995**
(21) Application number: 91830164.9
(22) Date of filing: 23.04.1991
(51) Int. Cl.: A61F 13/15

(54) **Absorbent element incorporating hydrogel, and article including said element**
Absorbierendes Element mit Hydrogel und dieses Element enthaltender Artikel
Elément absorbant avec hydrogel, et article contenant cet élément

(30) Priority: 30.04.1990 IT 6732390
(43) Date of publication of application: 06.11.1991
(73) Proprietor: ANGELINI RICERCHE S.P.A. - SOCIETA' CONSORTILE (or, briefly, "ANGELINI RICERCHE S.P.A."), 00040 Pomezia - Santa Palomba (Roma) (IT)
(72) Inventor: Palumbo, Gianfranco, I-65125 Pescara (IT)
(74) Representative: Bosotti, Luciano

(56) References cited:
- EP-A- 0 146 190
- EP-A- 0 325 416
- EP-A- 0 374 105
- EP-A- 0 388 372
- FR-A- 2 361 079
- FR-A- 2 618 984

## Description

### Field of the invention

The present invention relates to an absorbent element which is made of hydrophilic fibres, for example cellulose fibres, and may have a discontinuous layer of particles of a hydrogelling absorbent material on its surface which faces the user.

### DESCRIPTION OF THE PRIOR ART

Disposable absorbent articles such as babies' diapers, incontinence pads for adults, sanitary towels and similar products are well known and all have absorbent elements for absorbing and retaining body fluids.

Such absorbent elements, commonly known as pads, must be able to take up the liquid quickly and distribute it internally so as to prevent leakage; moreover, they must have a good capacity for retaining the fluids when subjected to the normal pressures of use.

Absorbent pads made of cellulose fibres derived from conifer wood have satisfactory characteristics as regards the liquid-absorption rate and are therefore able effectively to distribute the liquid within them, but are much less effective from the point of view of retention and may allow the liquid to flow back or to leak from the absorbent structure under the normal pressures of use.

One way of avoiding this problem may be considerably to increase the quantity of absorbent material in the pad so that the liquid does not flow back or leak under the normal pressures of use; however, the very hydrophilic nature of cellulose and its absorption mechanism mean that the surface of the absorbent pad tends to remain damp in any case and can thus cause the user to feel wet.

One attempt to solve the problem of the flowing back and leakage of liquid has been to mix particles of a hydrogelling absorbent material with the cellulose to increase the absorption and retention capacities of the absorbent element.

Hydrogelling materials, commonly known as superabsorbents, are polymers which can swell up and absorb large quantities of liquid, particularly water and, to a lesser extent, also body fluids.

They also have the particular property that they retain these fluids even under moderate pressure; because of these characteristics, their use in disposable absorbent articles has been proposed for some time.

In principle, the simple addition of superabsorbent material to the hydrophilic fibres makes it possible to produce a pad of conventional weight with a greater absorption capacity that can be achieved with the same quantity of hydrophilic fibres alone; conversely, the use of superabsorbent material and a smaller quantity of hydrophilic fibres enables a lighter and thinner pad to have the absorption capacity of a pad of conventional weight.

The good absorption capacity of superabsorbents, however, is not accompanied by an equally good absorption rate and this can adversely affect the performance of absorbent articles incorporating these substances.

In fact, superabsorbents can give rise to a phenomenon known in the prior art as "gel blocking": when a superabsorbent particle comes into contact with the liquid, its outer surface starts to absorb the liquid and swells up, preventing the liquid from passing into the particle; the liquid can only penetrate further into the still-dry core of the particle by a very slow diffusion mechanism.

This phenomenon can prevent full use from being made of the large absorption capacities of superabsorbent substances and, in structures in which superabsorbents are mixed uniformly with the hydrophilic fibres, may also involve a decrease in the absorption rate and consequently a greater chance of leakage.

In these structures, there is thus a marked decrease in the rate at which the liquid spreads into the absorbent pad.

Finally, although superabsorbent substances have the advantage, compared with cellulose fibres, that they retain absorbed liquids securely, the use of these substances simply mixed uniformly with cellulose fibres presents problems.

One of the many examples of absorbent structures constituted by mixtures of superabsorbents and cellulose fibres is provided by European patent application EP-A-122042 which proposes a very dense absorbent structure in an attempt to resolve the aforementioned problem.

European Patent application EP-A-254476 proposes a low-density region positioned in the absorbent pad so as to receive the liquid directly upon its release; this region constitutes a kind of temporary storage sump for the liquid which must then be absorbed into the denser surrounding regions of the pad.

A substantially different approach to the solution of the problem of liquid flow-back from that based on the use of superabsorbents in a mixture is offered in US Patent No. 3888256.

According to this patent, the superabsorbent is only on the upper surface of a conventional pad; the quantity used is such that, as the particles absorb and swell up, they interpenetrate each other to form a continuous layer which prevents the liquid from flowing back under normal conditions of use; however, this continuous layer also obstructs the absorption of any further quantities of liquid which may be released subsequently by the user.

A solution which aims to avoid the problems described is proposed in the present Applicant's European patent application No. EP-A-374105, in which an absorbent element of normal hydrophilic cellulose fibres has a discontinuous layer of hydrogelling absorbent material on its upper surface facing the user's body and , even when wet, it does not form a continuous impermeable layer such as to prevent the further penetration of liquid into the absorbent pad.

Another solution described in European Patent application No. EP-A-388372 also in the name of the present Applicant, proposes an absorbent pad including regions along its side edges in which hydrogelling absorbent material is distributed at a high concentration, either as a mixture or on the surface, in order to reduce the lateral leakage of liquid.

Also, FR-A-2 618 984, after which the preamble of Claim 1 was patterned, discloses an absorbent element wherein superabsorbent material is located in pouches adjacent the front and rear ends of an absorbent pad.

Although the solutions of the two above captioned European patents have been shown to be effective in providing an improved feeling of dryness, particularly in versions which are different according to the sex of the user, and better fluid retention as regards lateral leakage, there is still the problem of providing an absorbent structure which can make use of the good absorption and retention capacities of superabsorbents without incurring the disadvantages described above in connection with the use of superabsorbent materials in a mixture, and which preferably enables the quantity of hydrophilic fibres to be reduced so as to provide a thinner and lighter pad.

### Objects and summary of the invention

The object of the present invention is to improve the absorption and retention capacities of absorbent elements for use in disposable absorbent articles.

According to the present invention, this object is achieved by virtue of an absorbent element having the characteristics recited specifically in the characterising portion of Claim 1.

A further object of the invention is an absorbent article according to Claim 8.

In summary, the invention relates to an improved absorbent element which has two regions at its front and rear longitudinal ends in which the hydrogelling absorbent material is distributed in a mixture with the hydrophilic fibres.

### Detailed description of the invention

Further characteristics and advantages of the invention will become clear from the following description given purely by way of indicative, but non-limiting, example with reference to the appended drawings, in which:
Figure 1 is a plan view of a disposable diaper using an absorbent element according to the present invention,
Figure 2 is a sectional view of the diaper taken on the line II-II of Figure 1 (the longitudinal axis AA′),
Figure 3 is a plan view showing only the absorbent element of Figure 1, of which Figure 4 shows a particular distribution of the superabsorbent material in a mixture, and
Figure 5 is a section taken on the axis AA′ of an absorbent element according to the present invention, showing the element during the absorption of a certain quantity of liquid.

The absorbent elements of the present invention will be described with reference to their use in disposable absorbent articles; these articles are worn by the user in direct contact with the body to absorb body fluids and are then thrown away after a single use.

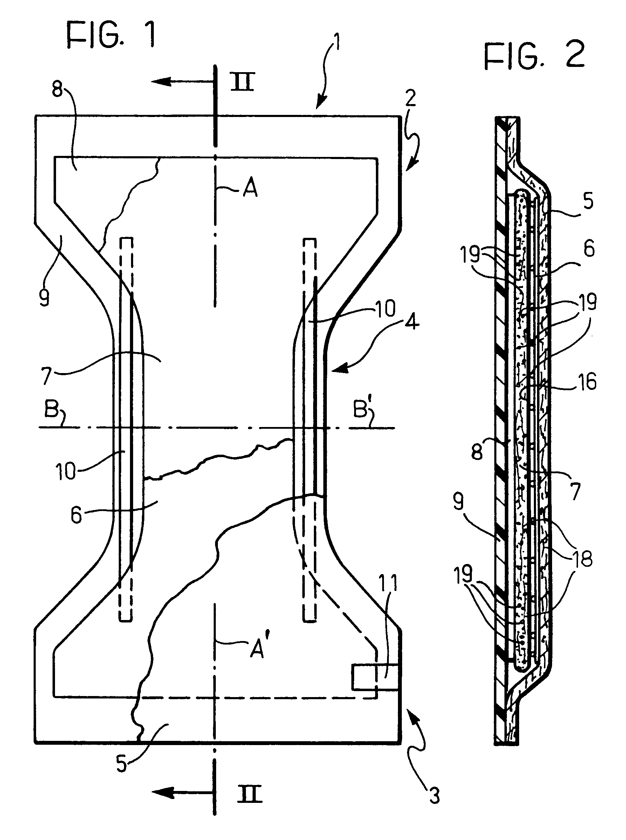

The disposable diaper shown in Figure 1 represents a preferred embodiment of an absorbent article according to the present invention but the present invention is also intended to be applied to other disposable absorbent articles, such as articles for the incontinent, sanitary towels and the like.

Figure 1 is a plan view of a diaper 1 in an extended configuration with some portions of its structure removed to show the construction of the diaper more clearly; in particular, the side of the diaper which comes directly into contact with the user is shown.

Figure 1 shows two end regions, a front end region 2 and a rear end region 3, which in use are positioned around the user's waist, and a central region 4 between them which is positioned in the crotch region; a longitudinal axis AA′ and a transverse axis BB′ are also shown.

The diaper comprises an upper layer 5 of non-woven fabric which is permeable to liquids and is intended to come directly into contact with the user's skin, a layer 6 of tissue which is preferably of the wet-strength type, immediately beneath the non-woven fabric , an absorbent element 7 according to the present invention, which is described further below, a second layer 8 of tissue which is preferably of the wet-strength type, an impermeable plastics sheet 9, and elastic elements 10 on both sides of the absorbent element 7 for effecting a seal around the user's legs in use; one of the two adhesive tabs 11 commonly used for fixing the diaper 1 around the user's waist is also visible on the rear region 3.

The sheet of non-woven fabric 5 and the plastics sheet 9 are of the same shape and size and correspond to the outline 1 of the whole diaper, whilst the two layers of tissue 6 and 8 are shaped like the absorbent element 7 between them.

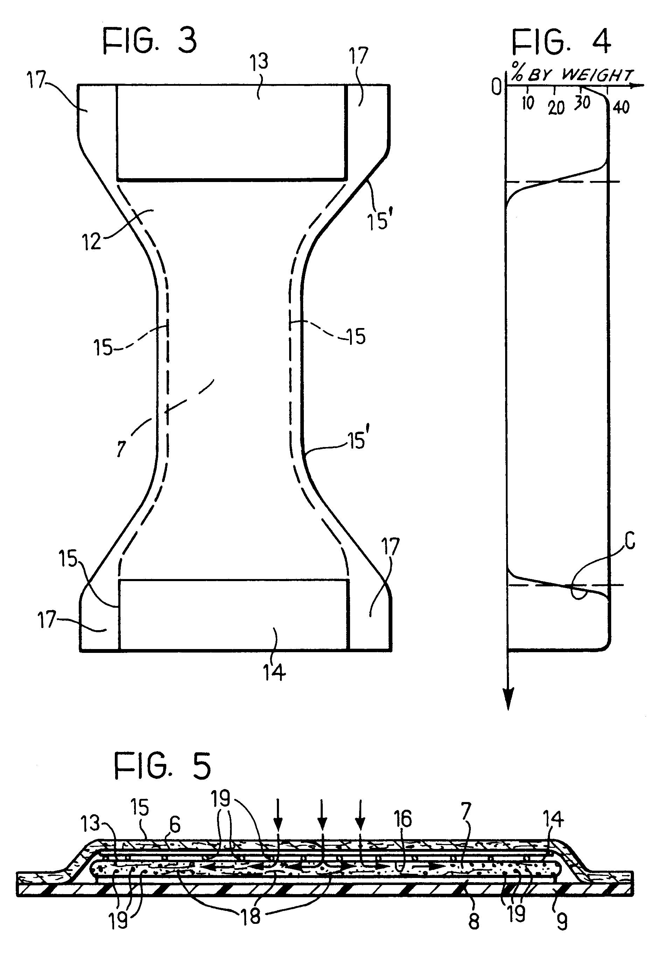

In the configuration shown, the absorbent element 7 has a conventional hourglass-shape, as can better be seen in Figure 3 where it is shown on its own; this drawing shows a main portion 12 which is generally affected by the distribution of the superabsorbent material and is of the same length as the element itself, including two regions 13 and 14 centred on the longitudinal axis AA′ at the front and rear ends 2, 3 of the pad respectively.

The main portion 12 does not necessarily include the entire upper surface 16 of the pad but, as in the embodiment illustrated, may exclude larger or smaller parts of the so-called ears 17 of the pad; thus, the longitudinal edges 15 of the main portion 12 of the pad may not coincide with the longitudinal edges 15′ of the pad, at least in the four end regions 17, since they are situated inwardly thereof.

In the preferred configurations the two end regions 13 and 14 are rectangular and their perimeters are entirely inside the edges of the main portion 12 of the pad; their width may be smaller or equal to that of the main portion 12 of the pad but, in any case, it cannot be smaller than the minimum width of the pad in correspondence with the central region 4; the regions 13 and 14 may both be of the same length but the front region 13 is preferably longer than the rear region 14.
The absorbent element 7 is made of hydrophilic fibres 18, such as, for example, cellulose fibres derived from conifer wood, and particles 19 of hydrogelling absorbent material.

Figure 2 shows how the particles 19 of superabsorbent material are distributed in the absorbent element 7 and are in a mixture with the cellulose fibres in the front and rear end regions 13 and 14; particles of hydrogelling absorbent material are preferably also distributed on the surface 16 of the absorbent element 7; the surface distribution involves the main portion 12 of the pad and is non-continuous.

Suitable hydrogelling absorbent materials may be inorganic or organic materials, such as cross-linked polymers wholly known in the art.

If, as it is preferable, the absorbent element has superabsorbent particles distributed both in a mixture in the two end regions 13 and 14 and on the surface of the main portion 12, the same type of superabsorbent material may be used for the mixture and for the surface distribution; however, the superabsorbent material in the mixture may be preferably characterised by a faster absorption rate than that of the superabsorbent material which is distributed on the surface.

The average size of the particles 19, that is, the weighted average of the smallest dimensions of the individual particles, may be between 100 and 800 microns, preferably between 300 and 600 microns.

The surface distribution of the superabsorbent particles 19 on the main portion 12 of the pad, when present, must be non-continuous, that is, such as to ensure that the particles are substantially isolated from each other before the absorption of liquid and that they remain so even after the swelling which results from the absorption, or at any rate that they do not form a continuous layer on the surface of the pad when they swell up.

In general, such a result is achieved by a surface distribution of particles 19 of the preferred dimensions at concentrations of between 5 g/m² and 70 g/m², preferably between 10 g/m² and 60 g/m².

In order to increase the overall absorption capacity of the absorbent element 7 of the present invention, the end regions 13 and 14 of the main portion 12 of the pad are characterised by the presence of a hydrogelling absorbent material distributed in a mixture with hydrophilic fibres; this is particularly important if the absorbent element is of the thin type and includes a smaller quantity of hydrophilic fibres than is used in a pad formed of fibres alone.

A suitable quantity of superabsorbent material simply mixed with hydrophilic fibres can provide a lighter element with a theoretical absorption capacity equal to that of a pad made entirely of a larger quantity of hydrophilic fibres; in order to avoid the problems described above in connection with the "gel blocking" phenomenon, the superabsorbent material is only present in a mixture in the front and rear end regions 13 and 14 of the pad.

Figure 3 shows a preferred configuration of this distribution; one can see therein the main portion 12 of the pad 7 including the two rectangular front and rear end regions 13 and 14 situated at the longitudinal ends of the pad 7, both of same width as the longitudinal ends of the main portion 12; measured along the longitudinal axis AA′, the front region 13 is preferably longer than the rear region 14.

The quantities of the particles of superabsorbent material and of the hydrophilic fibres in the two end regions 13 and 14 of the absorbent element 7 can conveniently be expressed as percentages by weight with reference to the portion of the absorbent element 7 comprised in the two end regions.

Although, in principle, the concentrations of the superabsorbent material may be different in the front and rear end regions 13 and 14 and may also vary longitudinally and transversely, in a preferred embodiment they are the same in both regions and are uniform throughout the regions, as shown in the graph of Figure 4 which shows the variation of the concentration C of the superabsorbent material in a mixture as a percentage by weight of the absorbent element along the longitudinal axis AA′ starting from the outer edge of the end region 13 (the origin of the abscissa).

The graph shows that the superabsorbent material is present in a mixture only in the front and rear end regions 13 and 14.

The concentration of the superabsorbent material in a mixture in each of the two end regions 13 and 14 starts at a maximum value and reduces gradually to zero towards the centre of the absorbent element but is uniform transversely.

The end regions 13 and 14 may contain between 5% and 70% by weight of superabsorbent material distributed in a mixture, and preferably between 20% and 50% by weight.

The length of each of the end regions may be between 5% and 30% of the total length of the pad; preferably, the length of the front region 13 is between 15% and 20% of the total length, whilst the length of the rear region 14 is between 10% and 15%.

Surprisingly, it has been found that the presence of a superabsorbent material in a mixture at these concentrations in the two end regions 13 and 14 of the pad brings considerable advantages in use and, in particular, provides good absorption and retention capacities combined with good distribution of the fluid in lighter, thinner absorbent elements, without incurring the limitations and disadvantages connected with the use of a superabsorbent material mixed uniformly with hydrophilic fibres.

This distribution of the superabsorbent material represents an apparent contradiction of what has been said hitherto as regards the absorption and distribution capacities of absorbent elements including superabsorbent substances in a mixture particularly with reference to the "gel blocking" phenomenon.

Although this phenomenon is not yet completely understood, in practice, it results in a slower rate of spread of the fluids into the pads containing hydrogelling material, as explained above.

The improved behaviour exhibited by the pads according to the present invention is due to the fact that the superabsorbent material in a mixture is localised particularly in the front and rear end regions 13 and 14 of the absorbent element.

As shown in Figure 5, the liquid is generally received in the central region of the pad; this central region contains no superabsorbent particles which might obstruct the spread of the liquid as a result of the "gel blocking" phenomenon and can therefore transfer the liquid quickly by capillarity to the peripheral regions of the absorbent element, and particularly towards the front and rear end regions 13 and 14 which are characterised by the presence of superabsorbent material in a mixture.

The ability of the hydrophilic fibres to transmit by capillarity the liquid from the reception point near the centre of the pad to the two end regions 13 and 14 could also be improved if the absorbent element were made slightly denser; this would further reduce the thickness of the pad.

The main characteristic of the end regions 13 and 14 is their overall absorption and retention capacities resulting from the presence of the superabsorbent material, whilst their capacity to transfer the liquid is less important since they are at the ends of the absorbent element.

Any limited "gel blocking" which may occur in the end regions, therefore, will not adversely affect the qualities of the absorbent element of the present invention but, paradoxically, may have a positive effect since it tends to keep the liquid in the two end regions which thus actually behave like storage regions for the liquid absorbed.

Once it has been received in the absorbent element, the liquid preferably tends to migrate towards the two end regions 13 and 14 of the pad and remains trapped there because of the superabsorbent particles distributed in a mixture; the central region of the pad including the region which, in use, is intended to receive the flow of liquid does not become saturated and thus retains a good residual distribution capacity, which is particularly useful for subsequent releases of liquid.

A diaper including an absorbent element according to the present invention will be described by way of non-limiting example.

The liquid-permeable upper layer is a spun-bonded non-woven fabric made of hydrophobic polypropylene fibres about 0.180 mm thick and weighing about 23.00 g/m².

The intermediate layers between the non-woven fabric and the absorbent element and between that element and the underlying plastics sheet are made of wet-strength tissue about 0.080 mm thick and weighing about 18.00 g/m²; the plastics sheet is an impermeable film of polyethylene about 0,025 mm thick and weighing about 24.30 g/m².

The hourglass-shaped absorbent element is made of cellulose fibres with an average density of about 10 cm³/g; the pad is 455 mm long and 290 mm wide at its front and rear ends and 135 mm wide in its central region; the overall weight of the cellulose is 28 g.

The main portion of the pad where the superabsorbent material is distributed is also hourglass-shaped and of the same length as the pad; the main portion is 210 mm wide at its front and rear end regions while in its central region is of the same width as the corresponding region of the pad; the end regions of the ears are excluded from the main portion.

The two end regions including the superabsorbent material distributed in a mixture are therefore 210 mm wide and are 70 mm long in the front region and 50 mm long in the rear region.

The superabsorbent material is constituted by granules of Favor Sab 922 produced by Chemische Fabrik Stockhausen and has an average particle size of between 300 and 600 microns; in the two end regions, the superabsorbent material is distributed uniformly in a mixture at a concentration of about 40% by weight with reference to the portion of the absorbent element comprised in those regions; as well as being distributed in a mixture in these end regions, it is also spread over the entire surface of the main portion at a concentration of about 30 g/m².

In use, the diaper performed well both from the point of view of its capacity to absorb and retain liquid and, in particular, from the point of view of its ability to transfer the liquid internally.

Naturally, the principle of the invention remaining the same, the details of construction may be varied widely with respect to those described and illustrated, without thereby departing from the scope of the present invention.

## Claims

1. An absorbent element (7) made of hydrophilic fibres, said absorbent element (7) including a central region (4) having a minimum width as well as two regions (13, 14) at its front and rear ends respectively, said front and rear end regions (13, 14) having a greater width than said minimum width and having associated therewith a hydrogelling absorbent material, characterised in that, in said front and rear end regions (13, 14) said absorbent material is distributed in a mixture with the hydrophilic fibres at a maximum concentration value, while said concentration value reduces gradually to zero towards the centre of the absorbent element.

2. An absorbent element according to Claim 1, characterised in that the concentration of the hydrogelling absorbent material distributed in a mixture with the hydrophilic fibres in the front and rear end regions (13, 14) is between 5% and 70% by weight, preferably between 20% and 50% by weight.

3. An absorbent element according to Claim 1 or Claim 2, characterised in that each of the front and rear end regions (13, 14) has a length between 5% and 30% of the absorbent element.

4. An absorbent element according to Claim 3, characterised in that the length of the front end region (13) is between 15% and 20% of the overall length of the absorbent element (7) and the length of the rear end region (14) is between 10% and 15% of the overall length of the absorbent element (7).

5. An absorbent element according to any one of the preceding claims, characterised in that the upper surface which is intended to face the user has a non-continuous layer of hydrogelling absorbent material (19) distributed at a concentration of between 5 g/m² and 70 g/m², preferably between 10 g/m² and 60 g/m².

6. An absorbent element according to any one of the preceding claims, characterised in that the hydrogelling absorbent material is preferably a polyacrylate.

7. An absorbent element according to any one of the preceding claims, characterised in that the hydrogelling absorbent material is in the form of particles with an average size of between 100 »m and 800 »m, preferably between 300 »m and 600 »m.

8. An absorbent article including a lower impermeable layer (9), an upper liquid-permeable layer (5), and an absorbent element (7) between the lower layer (9) and the upper layer (5), characterised in that the absorbent element (7) is formed according to any one of the preceding claims.

9. An absorbent article according to Claim 8 in the form of a disposable diaper for babies.

## Patentansprüche

1. Absorbierendes Element (7), hergestellt aus hydrophilen Fasern, wobei das absorbierende Element (7) einen Mittelbereich (4) mit einer Mindestbreite sowie zwei Bereiche (13, 14) an seinen vorderen bzw. hinteren Enden umfaßt; wobei die vorderen und die hinteren Endbereiche (13, 14) eine größere Breite als die Mindestbreite besitzen und ein ihnen zugeordnetes hydrogelisierendes, absorbierendes Material aufweisen,
**dadurch gekennzeichnet,** daß in den vorderen und hinteren Endbereichen (13, 14) das absorbierende Material in einem Gemisch mit den hydrophilen Fasern mit einem maximalen Konzentrationswert verteilt ist, wahrend der Konzentrationswert sich in Richtung der Mitte des absorbierenden Elements allmählich auf 0 reduziert.

2. Absorbierendes Element nach Anspruch 1, dadurch gekennzeichnet, daß die Konzentration des hydrogelisierenden, absorbierenden Materials, welches in einem Gemisch mit den hydrophilen Fasern in den vorderen und hinteren Endbereichen (13, 14) verteilt ist, zwischen 5 und 70 Gewichts-% beträgt, vorzugsweise zwischen 20 und 50 Gewichts-%.

3. Absorbierendes Element nach Anspruch 1 oder Anspruch 2, dadurch gekennzeichnet, daß jeder der vorderen und der hinteren Endbereiche (13, 14) eine Länge zwischen 5% und 30% des absorbierenden Elements besitzen.

4. Absorbierendes Element nach Anspruch 3, dadurch gekennzeichnet, daß die Länge des vorderen Endbereichs (13) zwischen 15% und 20% der Gesamtlänge des absorbierenden Elements (7) beträgt und daß die Länge des hinteren Endbereichs (14) zwischen 10% und 15% der Gesamtlänge des absorbierenden Elements (7) beträgt.

5. Absorbierendes Element nach einem der vorhergehenden Ansprüche, dadurch gekennzeichnet, daß die obere Oberfläche, welche dem Benutzer zugewendet werden soll, eine nicht durchgehende Schicht von hydrogelisierendem, absorbierendem Material (19) hat, das mit einer Konzentration zwischen 5g/m² und 70g/m² verteilt ist, vorzugsweise zwischen 10g/m² und 60g/m².

6. Absorbierendes Element nach einem der vorhergehenden Ansprüche, dadurch gekennzeichnet, daß das hydrogelisierende, absorbierende Material vorzugsweise ein Polyacrylat ist.

7. Absorbierendes Element nach einem der vorangehenden Ansprüche, dadurch gekennzeichnet, daß das hydrogelisierende, absorbierende Material in Form von Teilchen mit einer Durchschnittsgröße von zwischen 100»m und 800»m vorliegt, vorzugsweise zwischen 300»m und 600»m.

8. Absorbierender Gegenstand umfassend eine untere, undurchlässige Lage (9), eine obere, flüssigkeitsdurchlässige Lage (5) und ein absorbierendes Element (7) zwischen der unteren Lage (9) und der oberen Lage (5), **dadurch gekennzeichnet,** daß das absorbierende Element (7) gemäß einem der vorhergehenden Ansprüche ausgebildet ist.

9. Absorbierender Gegenstand nach Anspruch 8 in Gestalt einer Einweg-Windel für Babys.

## Revendications

1. Un élément absorbant (7) en fibres hydrophiles, ledit élément absorbant (7) comprenant une zone centrale (4) ayant une largeur minimale ainsi que deux zones (13, 14) respectivement à ses extrémités avant et arrière, lesdites zones d'extrémité avant et arrière (13, 14) ayant une largeur supérieure à ladite largeur minimale et ayant une matière absorbante formant un hydrogel qui leur est associée, caractérisé en ce que, dans lesdites zones d'extrémité avant et arrière (13, 14) ladite matière absorbante est répartie en mélange avec les fibres hydrophiles à une valeur de concentration maximale, ladite valeur de concentration diminuant progressivement jusqu'à zéro vers le centre de l'élément absorbant.

2. Un élément absorbant selon la revendication 1, caractérisé en ce que la concentration de la matière absorbante formant un hydrogel répartie en mélange avec les fibres hydrophiles dans les zones d'extrémité avant et arrière (13, 14) est comprise entre 5% et 70% en poids, de préférence entre 20% et 50% en poids.

3. Un élément absorbant selon la revendication 1 ou la revendication 2, caractérisé en ce que chacune des zones d'extrémité avant et arrière (13, 14) a une longueur comprise entre 5% et 30% de l'élément absorbant.

4. Un élément absorbant selon la revendication 3, caractérisé en ce que la longueur de la zone d'extrémité avant (13) est comprise entre 15% et 20% de la longueur totale de l'élément absorbant (7) et la longueur de la zone d'extrémité arrière (14) est comprise entre 10% et 15% de la longueur totale de l'élément absorbant (7).

5. Un élément absorbant selon l'une quelconque des revendications précédentes, caractérisé en ce que la surface supérieure qui est destinée à être orientée vers l'utilisateur à une couche non continue de matière absorbante formant un hydrogel (19) répartie à une concentration comprise entre 5 g/m² et 70 g/m², de préférence entre 10 g/m² et 60 g/m².

6. Un élément absorbant selon l'une quelconque des revendications précédentes, caractérisé en ce que la matière absorbante formant un hydrogel est de préférence un polyacrylate.

7. Un élément absorbant selon l'une quelconque des revendications précédentes, caractérisé en ce que la matière absorbante formant un hydrogel est sous la forme de particules avec une taille moyenne comprise entre 100 »m et 800 »m, de préférence entre 300 »m et 600 »m.

8. Un article absorbant comprenant une couche inférieure imperméable (9), une couche supérieure perméable aux liquides (5), et un élément absorbant (7) entre la couche inférieure (9) et la couche supérieure (5), caractérisé en ce que l'élément absorbant (7) est formé selon l'une quelconque des revendications précédentes.

9. Un article absorbant selon la revendication 8 sous la forme d'une couche jetable pour bébés.
